# EUROPEAN PATENT APPLICATION

(11) **EP 4 169 943 A1**
(43) Date of publication of application: **26.04.2023**
(21) Application number: 21218024.4
(22) Date of filing: 28.12.2021
(51) Int. Cl.: C07K 16/24, A61P 17/06

(54) **USE OF MONOCLONAL ANTI-IL1BETA ANTIBODY IN THE PREPARATION OF MEDICINE FOR TREATING/INHIBITING PSORIASIS**

(30) Priority: 25.10.2021 CN 202111238433
(71) Applicant: Sun, Liangdan, Hefei City, Anhui 230022 (CN)
(72) Inventor: SUN, Liangdan, Hefei City, Anhui Province, 230022 (CN); ZHEN, Qi, Hefei City, Anhui Province, 230022 (CN); LI, Bao, Hefei City, Anhui Province, 230022 (CN)
(74) Representative: Ipside

(57) **Abstract**

The present invention relates to the field of pharmaceutical technology for treating skin diseases, in particular, to the use of an agent in the preparation of a medicine for treating/inhibiting psoriasis. The agent in the present invention is aimed at the inhibition or promotion of a relevant signal in dsDNA-AIM2-Caspase1-IL1b signaling pathway, thus realizing the purpose of treating/inhibiting psoriasis. The agent can effectively inhibit the immune response caused by the inflammasome when used in the medicine for treating/inhibiting psoriasis, thus realizing the therapeutic effect on psoriasis and autoimmune diseases. The agent can also promote the inflammasome response, enhance the body's immune response, thus realizing the therapeutic effect on immunodeficiency diseases.

## Description

### Technical Field

The present invention relates to the field of pharmaceutical technology for treating skin diseases, in particular, to the use of an agent in the preparation of a medicine for treating/inhibiting psoriasis.

### Background Art

Psoriasis, also known as psora, is a common, chronic, and recurrent autoimmune skin disease. The appearances of scaly erythema and plaques in the affected area due to keratinocyte hyperproliferation are the main clinical manifestations of psoriasis. The course of the disease is long, and in some cases, psoriasis would never heal for almost a lifetime of a patient. This disease, commonly occurred in young and middle-aged people, has a relatively great impact on the physical health and mental status of patients. Current studies have shown that cytokine IL17A/F is an important effector in psoriasis. Biological agents targeting IL17A/F have a good therapeutic effect on psoriasis in clinical. However, relevant biological agents are expensive and need to be used continuously in the later period, the cost of treatment is high, and psoriasis cannot be eradicated. The affected area of patients with psoriasis is mainly the skin, and the level of IL17A/F in the circulatory system is not strongly correlated with the disease. Therefore, finding the source of IL17A/F in patients with psoriasis and finding the key factors that target the pathway of IL17A/F are of great significance for the effective treatment of patients with psoriasis.

### Summary of the Invention

In view of this, the purpose of the present invention is to provide the use of an agent in the preparation of a medicine for treating/inhibiting psoriasis. By research on psoriasis pathogenesis, the present inventors have identified the dsDNA-AIM2-Caspase1-IL1b signaling pathway in psoriasis which causes the occurrence or exacerbation of psoriasis. The present invention achieves the purpose of treating or inhibiting psoriasis by inhibiting or enhancing various relevant signals in this signaling pathway.

The agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

Further, the agent is selected from IL-1β monoclonal antibodies.

Further, the dosage form of the medicine for treating/inhibiting psoriasis is a dressing, an oral medicament, a subcutaneous injection, or an intravenous injection.

Further, the present invention also provides the use of an agent in the preparation of inhibitors for inhibiting the IL17 secretion by γδ T cells. The IL17 secretion activity of γδ T cells is particularly strong in the epidermis and dermis of patients with psoriasis. Therefore, IL17 secretion by γδ T cells can also be inhibited through the inhibition of the dsDNA-AIM2-Caspase1-IL1b signaling pathway.

The agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

Further, the purpose of the present invention is to provide the use of an agent in the preparation of a medicine for inhibiting the spread of skin tissue lesions. The dsDNA-AIM2-Caspase1-IL1b signaling pathway is also intensely expressed in the activity of skin tissue lesions *in vitro.*

The agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

Further, the purpose of the present invention is to provide the use of an agent in the preparation of an inhibitor for keratinocyte proliferation in skin tissue, and the agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

Further, the purpose of the present invention is to provide the use of an agent in the preparation of an inhibitor for AIM2 inflammasome response, and the agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

Further, the purpose of the present invention is to provide a method for inhibiting IL17 secretion by γδ T cells *in vitro.* The method comprises knocking out AIM2-expressing gene and/or IL1b-expressing gene and/or receptor IL1r-expressing in cells *ex vivo.* The method for knockout can use existing gene editing technology.

Further, the purpose of the present invention is to provide a method for inhibiting AIM2 inflammasome response *in vitro,* characterized in that the method comprises using at least one of the following agents for interference: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

Further, the purpose of the present invention is to provide a method for inhibiting keratinocyte proliferation in skin tissue *in vitro.* The method comprises using at least one of the following agents for interference: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

In the present invention, the term "comprise" is an open expression, that is, includes the content specified in the present invention, but does not exclude other aspects.

In the present invention, the term "functional analogue" refers to substances with similar functions, and their structures are not limited to the same, as long as they have the same or similar functions.

In the present invention, the term "agonist" refers to a substance or drug that binds to the receptor of a certain biologically active substance and exhibits the effect of the active substance.

In the present invention, the term "antagonist" refers to a class of substances that can bind to a receptor but do not have intrinsic activity. Antagonists are divided into competitive antagonists and non-competitive antagonists.

In the present invention, the term "expression promoting agent" refers to a substance that can promote the expression of a certain protein or factor.

In the present invention, the term "expression inhibitor" refers to a substance that can inhibit the expression of a certain protein or factor.

In the present invention, the term "production inhibitor" refers to a substance that can inhibit the production of a certain substance.

In the present invention, the term "biological activity inhibitor" refers to a substance capable of inhibiting the biological activity of a certain substance.

### The beneficial effects of the present invention

The agent provided by the invention can be used in the preparation of a medicine for treating/inhibiting psoriasis and can effectively inhibit the immune response caused by inflammasomes, thus realizing the therapeutic effect on psoriasis and autoimmune diseases. The agent can also promote the inflammasome response, enhance the body's immune response, and thus play a role in the treatment of immunodeficiency diseases.

### Brief Description of the Drawings

Figure 1 shows the expressions of related proteins in the control group and the psoriasis group by immunohistochemistry/fluorescence detection.
Figure 2 shows the expressions of related proteins in the control group and the psoriasis group by Western.
Figure 3 shows the detection of IL1b level in a clinical serum sample.
Figure 4 shows the keratinocyte single cell sequencing of the skin tissue lesion of a patient with psoriasis.
Figure 5 shows the nuclear dsDNA content of the skin tissue lesion in normal people and a patient with psoriasis.
Figure 6 shows the skin of a mouse model of psoriasis induced by imquimod.
Figure 7 shows the detection of dsDNA-positive cells in the cytoplasm of the skin lesion by the Tunel method.
Figure 8 shows the cell after different concentrations of dsDNA being transferred into the skin keratinocyte line HaCaT.
Figure 9 shows the protein expression of downstream signaling pathway at different concentrations of dsDNA by Western.
Figure 10 shows the protein expression curve of downstream signaling pathway under the same concentration of dsDNA.
Figure 11 shows the results of human and mouse primary keratinocyte lines and HaCaT tool keratinocytes stimulated with oligodAT and IL17.
Figure 12 shows the flow cytometry after co-culture of dsDNA in exosomes and PBMC.
Figure 13 shows the flow cytometry of PI positive cells in normal people and a patient with psoriasis.
Figure 14 shows the dsDNA content of normal people and a patient with psoriasis.
Figure 15 shows an image of the relationship between free dsDNA in serum and dsDNA in exosomes.
Figure 16 shows the flow cytometry after PBMC being transfected with dsDNA (oligo dA-T) .
Figure 17 shows the relative changes of dsDNA in exosomes released by endothelial cells stimulated by cytokines TNFA and IL17.
Figure 18 shows the curve over time of dsDNA in exosomes released by endothelial cells stimulated by cytokines TNFA and IL17.
Figure 19 shows the expression of GSDM family genes in a clinical sample and a psoriasis skin lesion and a normal skin in a mouse model via expression profile.
Figure 20 shows the experiment of RNAi interference for GSDM.
Figure 21 shows the effect of different weights of imiquimod on psoriasis in a mouse.
Figure 22 shows the psoriasis in an IMQ mouse after targeted knockout of the Aim2 and IL1b genes of the Aim2 pathway.
Figure 23 shows the relationship between the skin lesion area and severity index of psoriasis in an IMQ mouse and time after targeted knockout of the Aim2 and IL1b genes of the Aim2 pathway.
Figure 24 shows the signal expression of the aim2 pathway in an IMQ mouse after targeted knockout of the Aim2 and IL1b genes of the Aim2 pathway.
Figure 25 shows the psoriasis of an IMQ mouse-oligo dAT after targeted knockout of the Aim2 and IL1b genes of the Aim2 pathway.
Figure 26 shows the spleen after injection of IL-1b into the ears of mice.
Figure 27 shows the psoriasis skin lesion area and severity index after injection of IL-1b into the ears of mice.
Figure 28 shows the phenotype of an imq mouse model after the use of IL-1b antibody.
Figure 29 shows the phenotype of a mouse induced by imiquimod after KO of the gene of IL1b receptor IL1r.
Figure 30 shows the flow cytometry of IL17 secretion by γδ T cells in an induced mouse model with psoriasis.
Figure 31 shows the fluorescence detection of IL17 secretion by γδ T cells in different mouse models.
Figure 32 shows the flow cytometry of IL17 secretion by γδ T cells in different mouse models.
Figure 33 shows the flow cytometry of IL17 secretion by γδ T cells in different mouse models.
Figure 34 shows a schematic diagram of the *in vitro* stimulation process after co-culture of mouse keratinocytes and immune cells.
Figure 35 shows the content of IL17 in mouse supernatant after *in vitro* stimulation.
Figure 36 shows the immunofluorescence of keratinocytes.
Figure 37 shows the signal expression of AIM2 pathway in different mouse models.
Figure 38 shows the psoriasis skin lesion area and severity index of a clinical mouse skin sample.
Figure 39 shows the flow cytometry of keratinocytes and IL1B expression.
Figure 40 shows the results of flow cytometry of immune cells in the epidermis and dermis.
Figure 41 shows the immunofluorescence detection of immune cells in the epidermis and dermis.
Figure 42 shows the expression of IL17 in immune cells in the epidermis and dermis.
Figure 43 shows the transcriptome sequencing.
Figure 44 shows the detection of DNA methylation level.
Figure 45 shows the ATAC sequencing.
Figure 46 shows the effect of AIM2 E32K mutant on psoriasis.

### Detailed Description of Embodiments

The examples given are to better illustrate the present invention, but the content of the present invention is not limited to the examples given. Therefore, the non-essential improvements and adjustments made by those skilled in the art to the examples based on the above-mentioned content of the invention still belong to the protection scope of the present invention.

### Example 1 Verification of dsDNA-AIM2-Caspase1-IL1b pathway activation in skin lesions of patients with psoriasis

Our previous research found that the free dsDNA in the circulatory system and skin of a patient with psoriasis increased significantly. In order to detect the effect of this change on the body, this example of the present invention tested its downstream receptor AIM2 and effector pathways, and the results are shown in Table 1 below. The RNA-seq results of the inventors and the European population both showed that the dsDNA-AIM2-Caspase1-IL1b pathway was activated.

**Table 1 dsDNA-AIM2-Caspase1-IL1b pathway related protein expression**

| Gene | N2C | P2N | P2C | RankP | aseMedia | ontMedia | FC |
|---|---|---|---|---|---|---|---|
| AIM2 | 1 | 5.00E-05 | 5.00E-05 | 2.05E-29 | 1.29 | 0.20 | 6.41 |
| CASP1 | 0.46805 | 0.001 | 0.06295 | 3.96E-18 | 28.87 | 19.57 | 1.48 |
| CASP4 | 0.248 | 5.00E-05 | 5.00E-05 | 3.58E-28 | 56.22 | 30.18 | 1.86 |
| CASP5 | 0.248 | 5.00E-05 | 5.00E-05 | 3.79E-26 | 0.70 | 0.07 | 9.82 |
| IL1b | 0.71135 | 5.00E-05 | 5.00E-05 | 1.06E-22 | 2.82 | 0.42 | 6.77 |
| PYCRAD | 0.8407 | 5.00E-05 | 5.00E-05 | 9.72E-22 | 100.89 | 50.21 | 2.01 |
| MLKL | 0.2779 | 5.00E-05 | 5.00E-05 | 2.70E-23 | 4.83 | 2.39 | 2.02 |

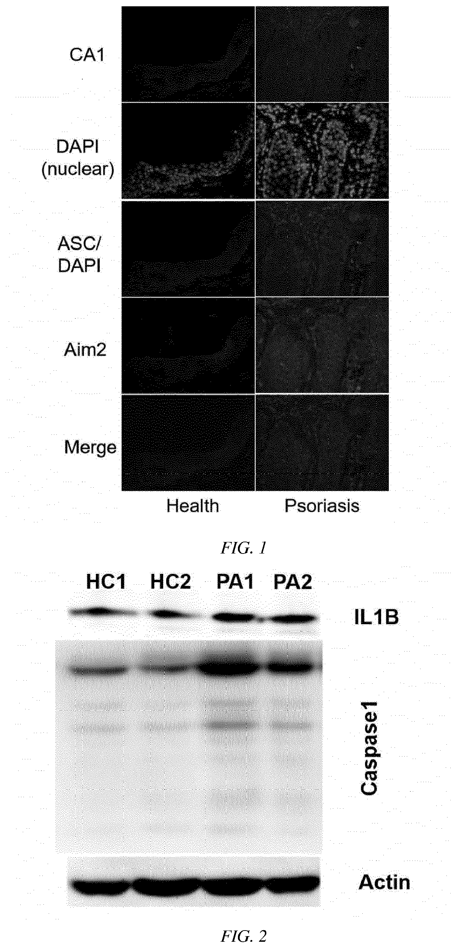

The protein levels were detected by Western and immunohistochemistry/fluorescence. The results are shown in Figure 1 and Figure 2. It can be seen that the protein expression of AIM2-Caspase1-IL1b pathway in psoriasis lesions is up-regulated.

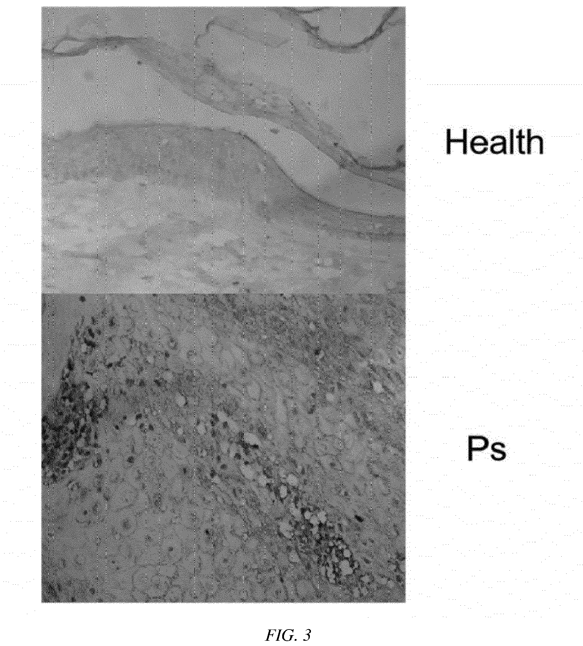

The IL1b level in a clinical serum sample was detected, the level of IL1b in the serum of a patient with psoriasis and normal people was compared. The results in Figure 3 show that the level of IL1b in the patient with psoriasis is significantly higher than that in normal people.

### Example 2 Verification by single-cell sequencing

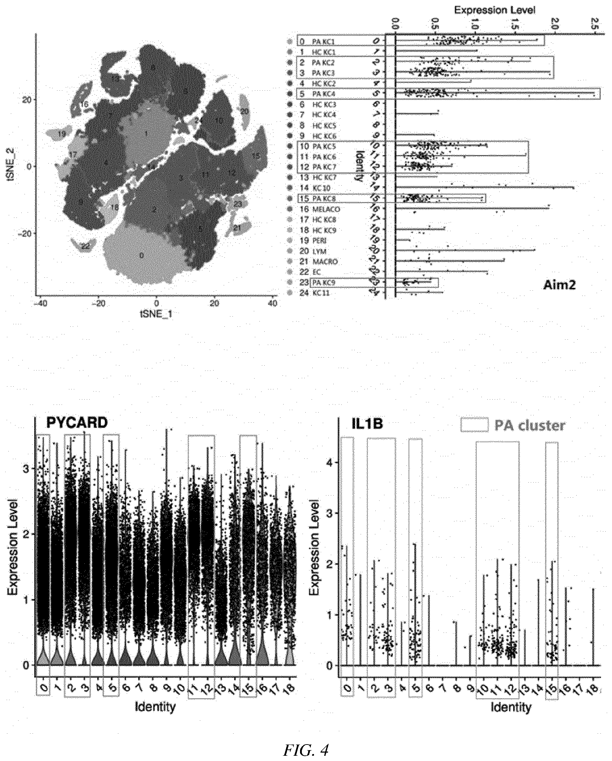

Single-cell sequencing of keratinocytes in the skin lesions of patients with psoriasis was performed. The results in Figure 4 show that the AIM2 pathway of multiple populations of keratinocytes in the skin lesions of the patients with psoriasis are specifically activated, while the AIM2 pathway in normal human skin is in an inactive state.

### Example 3 Verification by nuclear dsDNA detection in skin lesions

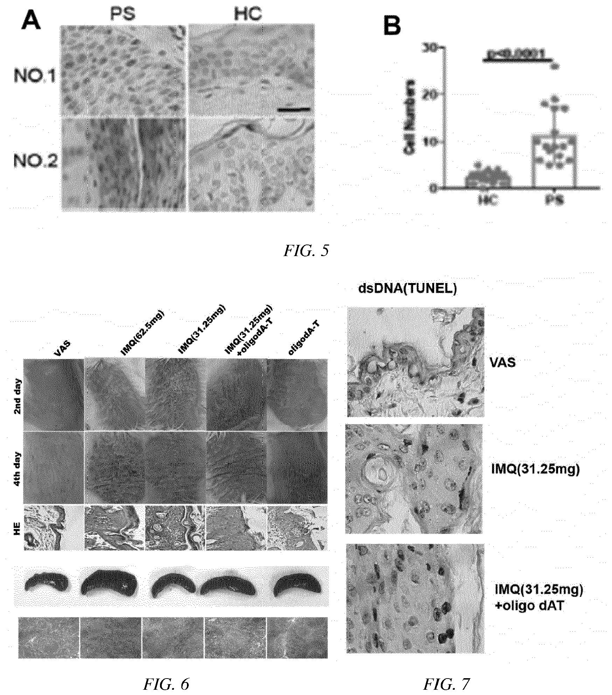

The nuclear dsDNA in the skin lesions of a patient with psoriasis and normal people was detected. The results are shown in Figure 5. The nuclear dsDNA content in the skin lesions in the patient with psoriasis is higher than the nuclear dsDNA content in normal skin.

### Example 4 Verification by skin tissue detection

In order to verify the experimental results of clinical samples, this example of the present invention used the classic imquimod-induced psoriasis mouse model (hereinafter referred to as imi mouse) to detect its skin tissue, and the detection is shown in Figure 6.

The number of dsDNA positive cells in the cytoplasm in the skin lesions was found to be significantly increased by the Tunel method (the results are shown in Figure 7), showing that the expression of dsDNA-AIM2-Caspase1-IL1b pathway in the psoriasis mouse model was increased, and the severity of skin lesions was positively correlated with the protein expression of the pathway.

### Example 5 Verification of the effect of free dsDNA in keratinocytes on cells

In the clinical sample detection in Example 4, it was found that the free dsDNA content in the keratinocytes in the skin lesions of a patient with psoriasis was significantly higher than that of the control skin.

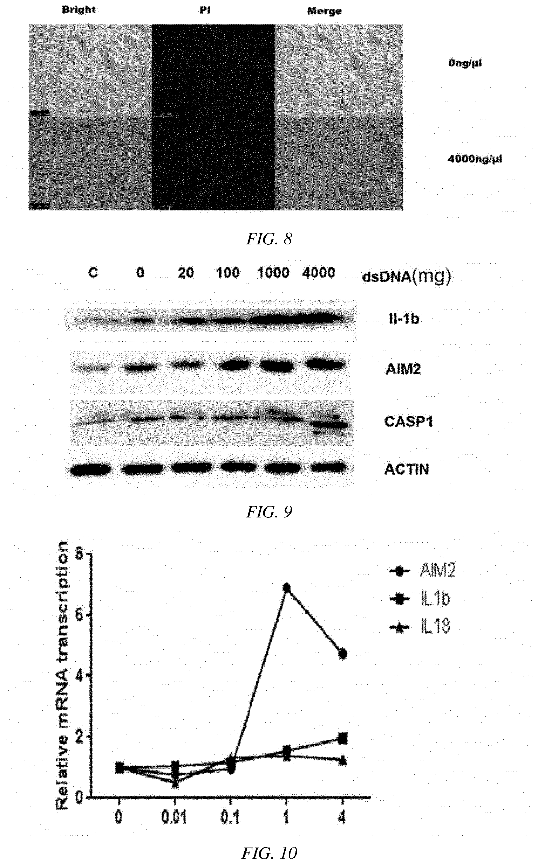

dsDNA can activate the dsDNA-Aim2 pathway in keratinocytes. In order to detect the effect of free dsDNA in keratinocytes on cells, this example of the present invention used classic lipfectine3000 to package different concentrations of dsDNA, the packaged dsDNA was transferred into the skin keratinocyte cell line HaCaT, and then the signal expression in downstream pathways of dsDNA was detected. The results in Figure 8, Figure 9 and Figure 10 show that dsDNA can activate the dsDNA-Aim2 pathway and induce keratinocyte pyroptosis, mainly releasing the cytokine IL1b. Lipofectamine3000 was diluted with DMEM and blended thoroughly. Oligodat was diluted with DMEM and P3000 was added and blended thoroughly. The prepared lipofectamine3000 was added to the prepared equal volume of P3000, blended thoroughly, and incubated at room temperature for 5 minutes. The prepared transfection reagent was added to the cell culture medium as needed.

### Example 6 Verification of the relationship between AIM2 pathway and IL17

IL17 aggravates keratinocyte pyroptosis and IL1b release induced by dsDNA. IL17 is an important clinically relevant cytokine for psoriasis. The inventors found that the dsDNA-AIM2-IL1B pathway was activated in clinical samples, animal models, and keratinocyte models.

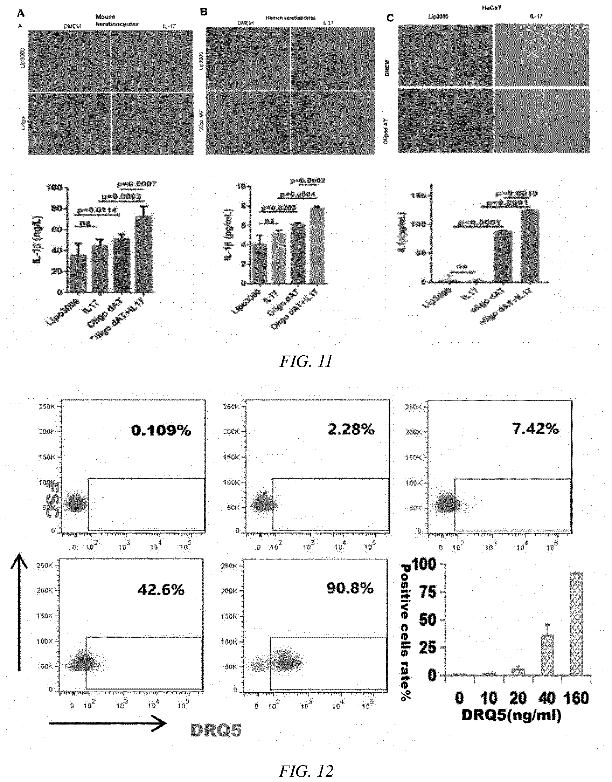

In order to identify the relationship between the AIM2 pathway and IL17, this example of the present invention used human and mouse primary keratinocyte lines and HaCaT tool keratinocyte lines stimulated with oligodAT, IL17, a combination of oligodAT and IL17, respectively. The stimulation results are shown in Figure 11. The results show that IL17 can significantly enhance and amplify the stimulating effect of oligodAT, including the occurrence of pyroptosis and the release of IL1b. The results further confirm the important role of the dsDNA-AIM2 pathway in the pathogenesis and exacerbation of psoriasis.

### Example 7

In this example of the present invention, the dsDNA in the isolated exosomes was labeled with DRQ5, and then co-cultured with PBMC. The culture results are shown in Figure 12, and the results show that the exosomes can effectively bring dsDNA in the body into target cells.

### Example 8

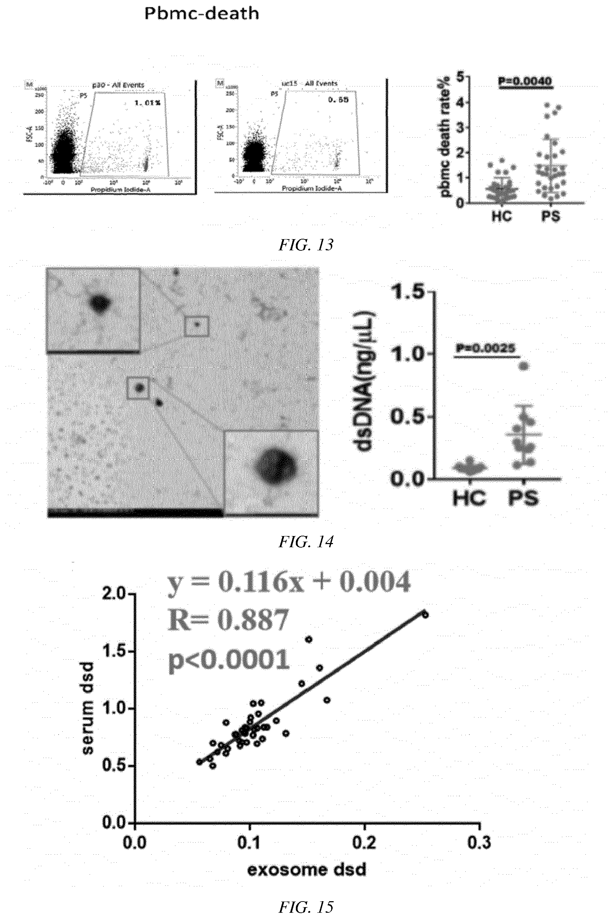

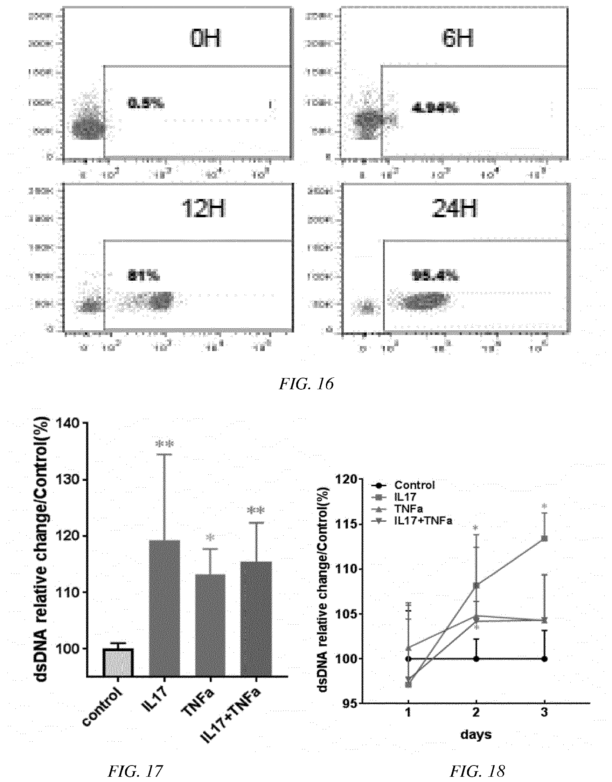

Both apoptosis and necrosis of PBMC release dsDNA. Therefore, this example of the present invention used flow cytometry to detect the death of PBMC in normal people and a patient with psoriasis. The results show that the PI-positive cells of PBMC in the patient with psoriasis are significantly higher than those in normal people (as shown in Figure 13); Figure 14 shows that the isolation and identification of serum exosomes showed that the dsDNA content in a patient was significantly higher than that in normal people and the free dsDNA in serum and the content of dsDNA in exosomes were significantly linearly correlated (as shown in Figure 15). Then dsDNA (oligo dA-T) was used to transfect PBMC. The results in Figure 16 show that dsDNA entering the cytoplasm can induce aggravation of psoriasis.

### Example 9

The dsDNA in exosomes released by endothelial cells stimulated by cytokines TNFA and IL17 was detected. The results are shown in Figure 17 and Figure 18. The cytokines TNFA and IL17 significantly stimulated the dsDNA content in exosomes released by endothelial cells.

### Example 10

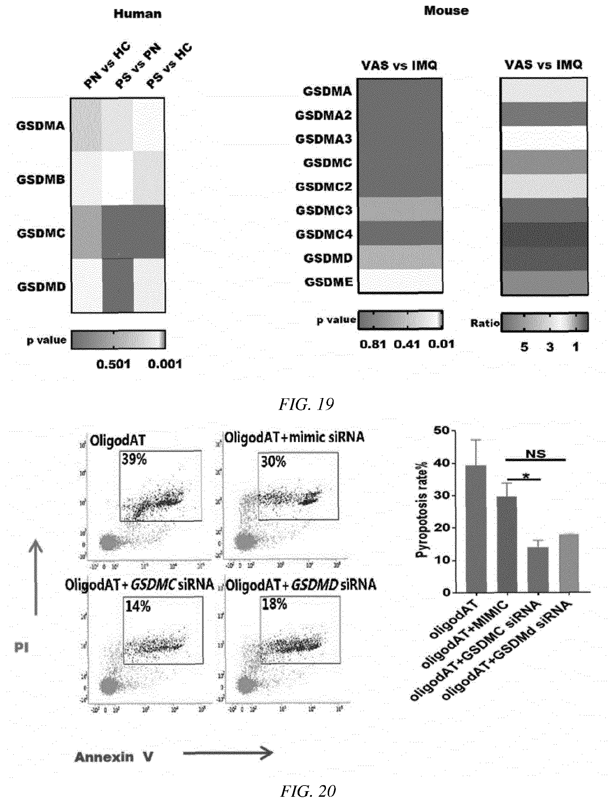

This example of the present invention used expression profile to detect the expression of GSDM family genes in a clinical sample and a psoriasis skin lesion and a normal skin in a mouse model. The results are shown in Figure 19. The RNAi interference experiment for GSDM was performed. The results in Figure 20 show that gsdmc plays a more important role in the activation of the Aim2 pathway in HaCaT cells.

### Example 11

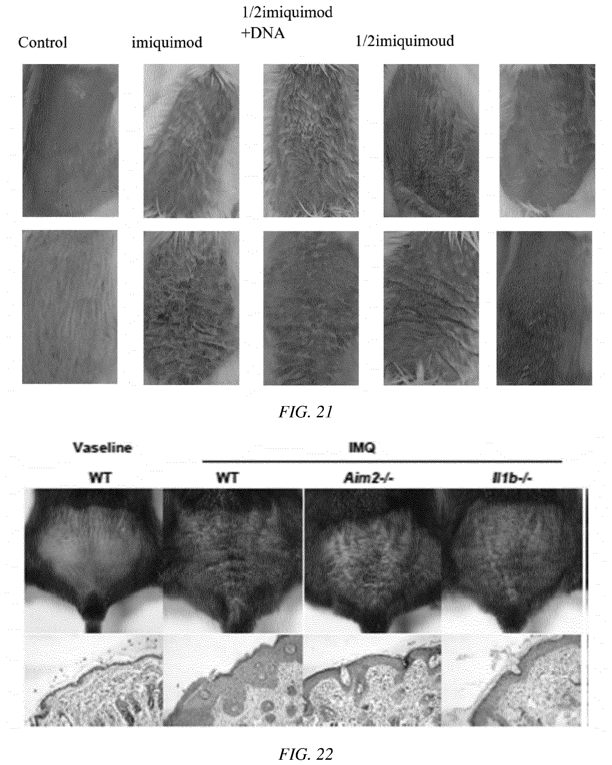

In this example of the present invention, a dsDNA (*in vivo*/*in vitro* administration) aggravation experiment was performed. The results are shown in Figure 21. Aggravated and prolonged psoriasis phenotype is observed in imq model + lip- (oligo dat), and the psoriasis response is the most obvious when using lip to package dsDNA to deliver the dsDNA into cells. (PASI and HE/tunel: dsDNA A. in cytoplasma B.higher level in ps skin lesion/western: aim2 pycard caspase1) The detection of pathway-related genes indicated that the pathway was activated.

### Example 12

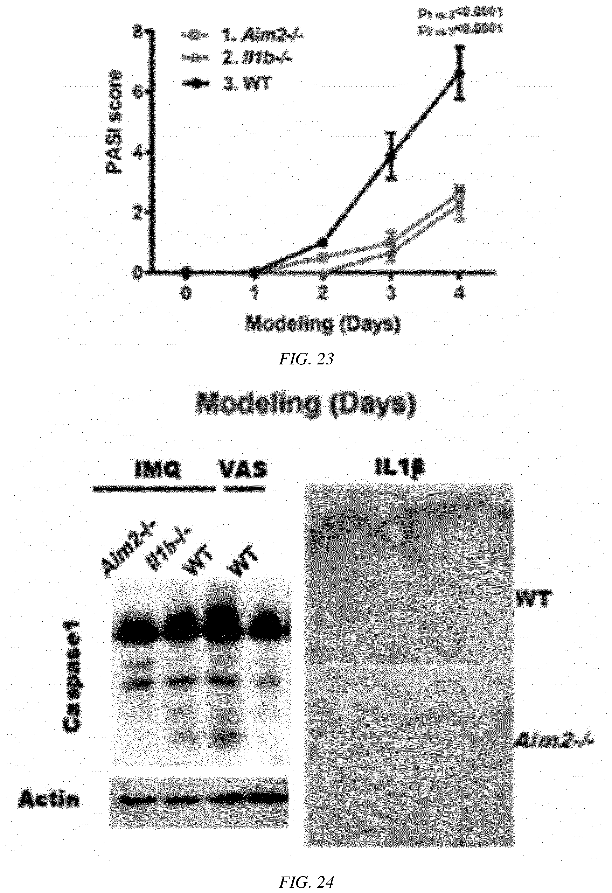

In this example of the present invention, an IMQ mouse experiment and a dsDNA aggravation experiment after targeted knockout of the Aim2 and IL1b genes of the Aim2 pathway were performed. The results are shown in Figure 22, Figure 23, Figure 24, and Figure 25. The imq-induced psoriasis-like inflammatory response in Aim2 KO mouse was mild and recovered quickly (PASI and HE/tunel: dsDNA A. in cytoplasma B.higher level in ps skin lesion/western: aim2 pycard caspase1), indicating that the Aim2-mediated inflammasome pathway played an important role in the maintenance and deterioration of the psoriasis response.

### Example 13

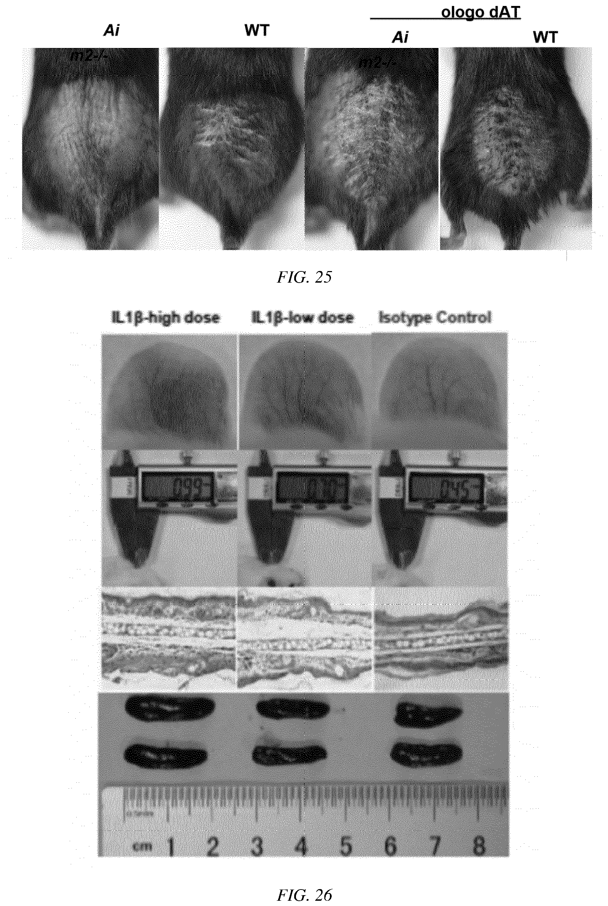

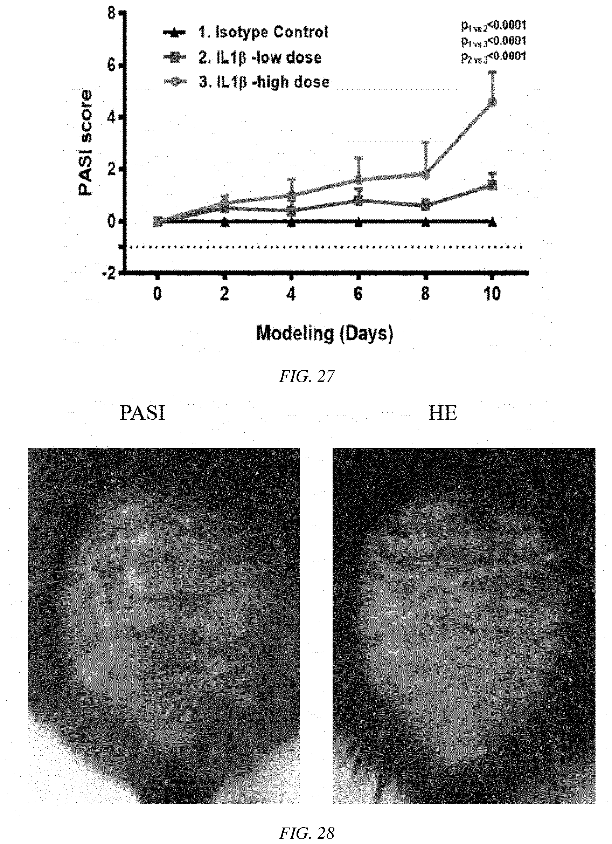

dsDNA-AIM2 finally released the cytokine IL-1b to act on the body. Clinical tests also showed that the skin tissue lesions and serum of psoriasis and the results of animal experiments showed that IL-1b was significantly increased in psoriasis. In order to identify whether the cytokine IL-1b alone can cause psoriasis-like response in a mouse, this example of the present invention used IL-1b to inject ears of the mouse so as to cause psoriasis-like skin phenotype, spleen enlargement and pathological changes. The results are shown in Figure 26 and Figure 27.

### Example 14

Because dsDNA-AIM2 finally released the cytokine IL-1b to act on the body to aggravate psoriasis, if IL-1b played an important role in imiquimoud-induced mouse psoriasis phenotype, the phenotype would be weakened after reduction of IL-1b activity. For this reason, this example of the present invention used an IL-1b antibody to neutralize IL-1b biological activity. The experimental results are shown in Figure 28. The results show that the 20x antibody can effectively alleviate the phenotype of the imq mouse model and achieve early recovery (PASI and HE).

### Example 15

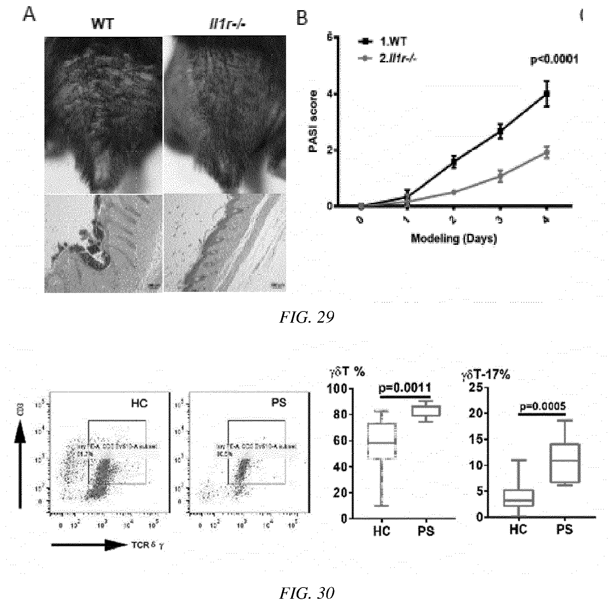

In order to verify the important role of the signaling pathway of the cytokine IL1b released after activation of this pathway in the pathogenesis of psoriasis, this example of the present invention knocked out the gene of mouse IL1b receptor IL1r, and again imiquimod was used for induction and the phenotype change was observed. The results in Figure 29 show that the phenotype after knockout of the gene of IL1b receptor IL1r is the same as that after AIM2 and IL1B KO, and the psoriasis phenotype is significantly alleviated.

### Example 16

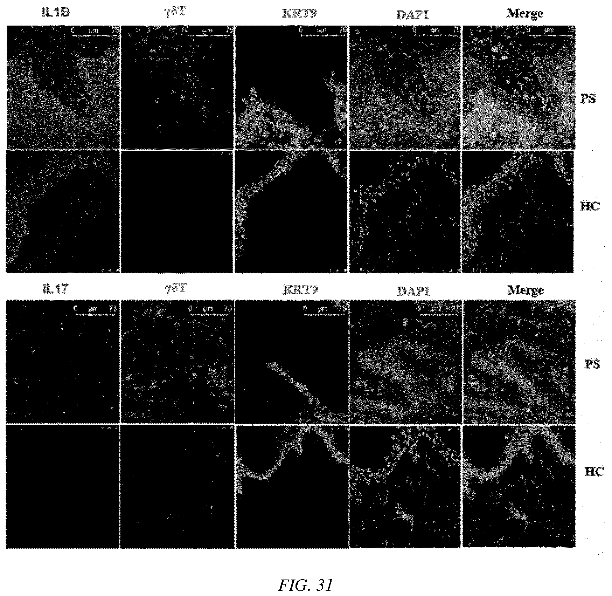

IL17 is an important pathogenic factor for psoriasis. Studies have shown that γδ T cells are an important source of skin IL17, and are the most relevant T cell for psoriasis. This example of the present invention was to find the effect of dsDNA-AIM2-IL1b activation in the skin on the IL17 secretion by γδ T cells, and the IL17 secretion by γδ T cells was detected on the psoriasis-induced mouse model and after knockout of genes of AIM2, IL1b and IL1b receptor IL1r, respectively. The results in Figure 30, Figure 31, Figure 32, and Figure 33 show that the main source of IL17 in the skin is γδ T cells, and the IL17 secretion by γδ T cells is significantly reduced after the pathway is blocked.

### Example 17

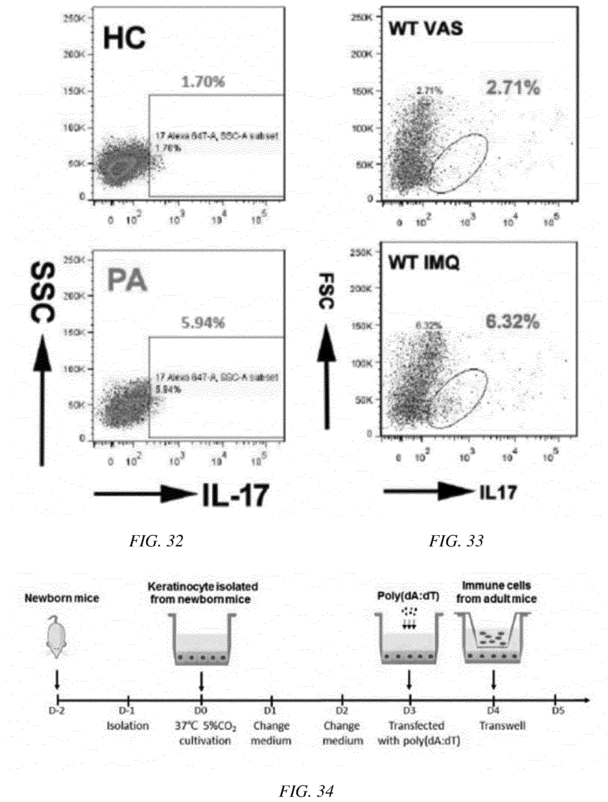

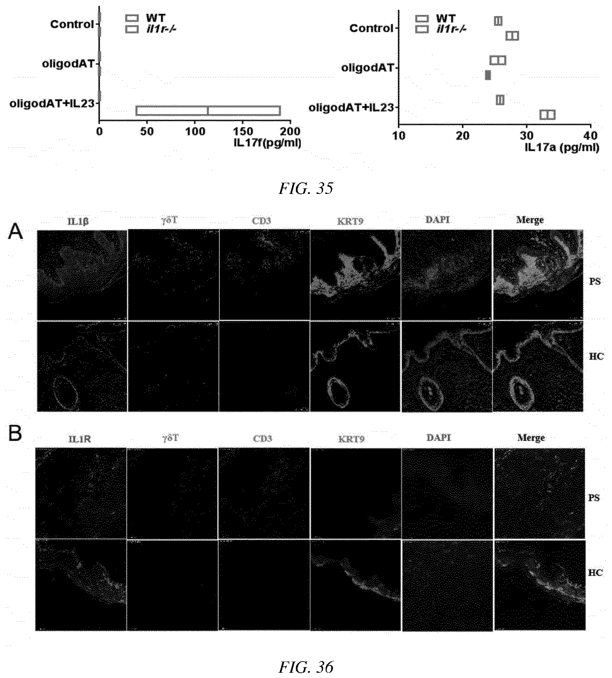

Transwell experiment showed that activation of the dsDNA-AIM2-ILIB pathway mainly affected the development of γδT-17. The mouse keratinocytes and immune cells were isolated for co-culture, and oligodAT and IL23 were added exogenously for *in vitro* stimulation (as shown in Figure 34). After the experiment, the content of IL17 in the supernatant was detected. This example of the present invention detected two cytokines A and F related to IL17 and psoriasis, respectively. The results are shown in Figure 35. The results show that the IL1 signaling pathway is an essential factor for IL17A/F secretion and mainly stimulates the production of IL17F.

**Example 18** Keratinocytes in skin tissue are the main source of IL1b, and the IL1B receptor system of the keratinocyte system is inhibited during the occurrence of psoriasis, while the IL1B receptor system of the immune cell system is activated.

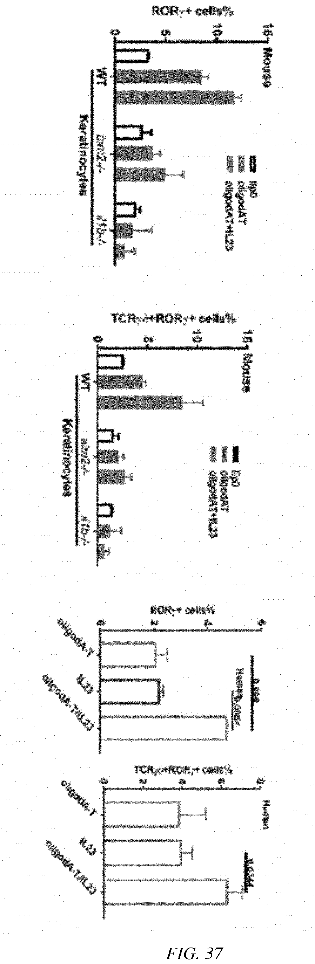

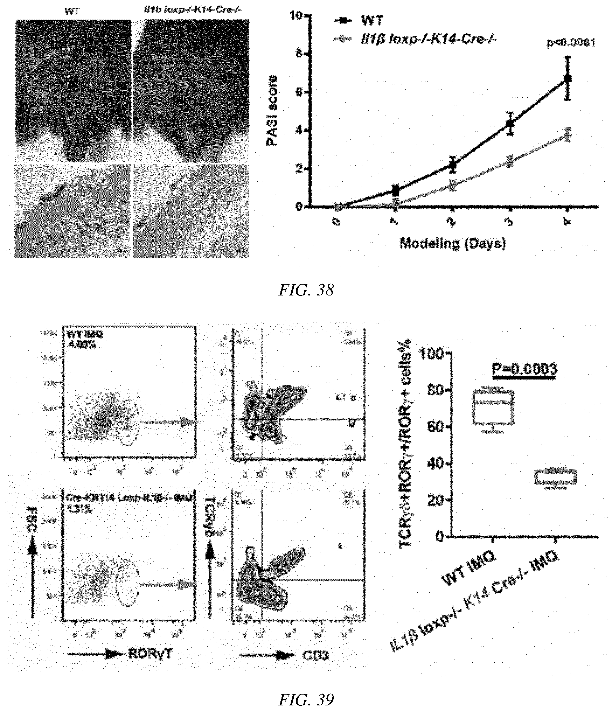

In Example 16, immunofluorescence co-labeling reveals that IL1b is mainly located in keratinocytes. In order to determine the role of keratinocyte-derived IL1b in the pathogenesis of psoriasis, in this example of the present invention, relevant detections were carried out through *in vitro* experiments, and the results are shown in Figure 36, Figure 37, Figure 38, and Figure 39. Immunofluorescence multi-labeling: the collected clinical skin samples were rinsed with PBS and then put into 30% sucrose solution and dehydrated in a refrigerator at 4 °C. After the skin tissue sinked to the bottom of the fixed container, a freezing microtome was used to obtain a section with a thickness of 20 microns. Keratinocyte antibodies, lymphocyte antibodies and TCRγ antibodies with different fluorescent labels were used for multiple fluorescent staining, and finally DAPI was used for nuclear labeling. The slides were mounted with an anti-quenching agent and a laser confocal microscope was used for observation and photography. Interaction of keratinocytes and immune cells *in vitro:* the extracted human skin keratinocytes and mouse skin keratinocytes with different genotypes were cultured in the lower layer of the transwell chamber. Different stimuli were added. The isolated human primary skin lymphocytes and wild-type mouse skin lymphocytes were added to the upper layer. After co-culture, the supernatant and cells were collected for related detection.

### Example 19 Verification of γδT-17/RORγT migration to the epidermis

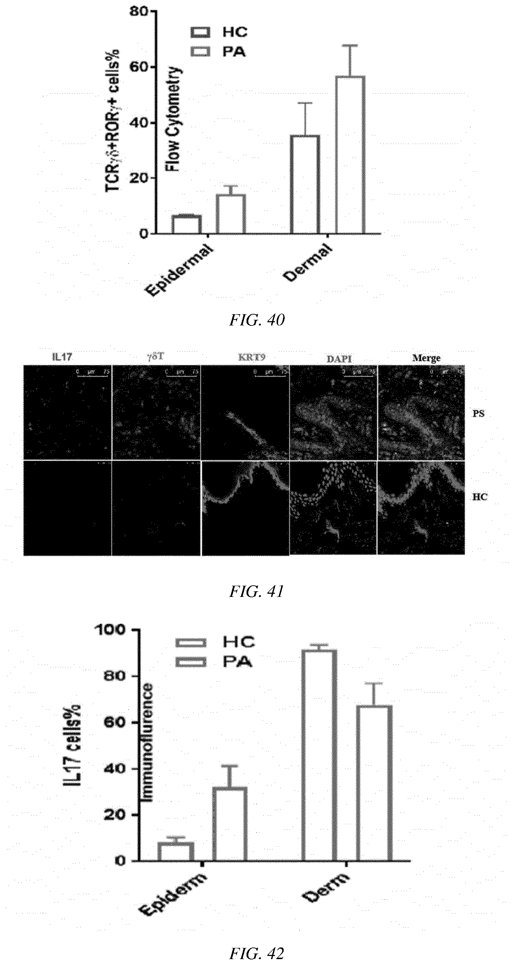

Flow cytometry was performed by isolating immune cells from the epidermis and dermis. It was found that γδT-17/RORγT in the epidermis of a patient with psoriasis and a mouse increased significantly (as shown in Figure 40). The results of immunofluorescence detection also showed that positive cells in the epidermis increased (as shown in Figure 41 and Figure 42). These results show that the migration of γδT-17/RORγT to the epidermis is a pathological sign of psoriasis, would also enhance the effect of the dsDNA-AIM2 pathway in the keratinocytes, accelerate the release of cytokine IL1B, and accelerate the formation of circulatory effect of pathological dsDNA-AIM2-IL1B-IL17 in psoriasis.

### Example 20 Significant correlation of dsDNA-AIM2 pathway with psoriasis at multiple levels

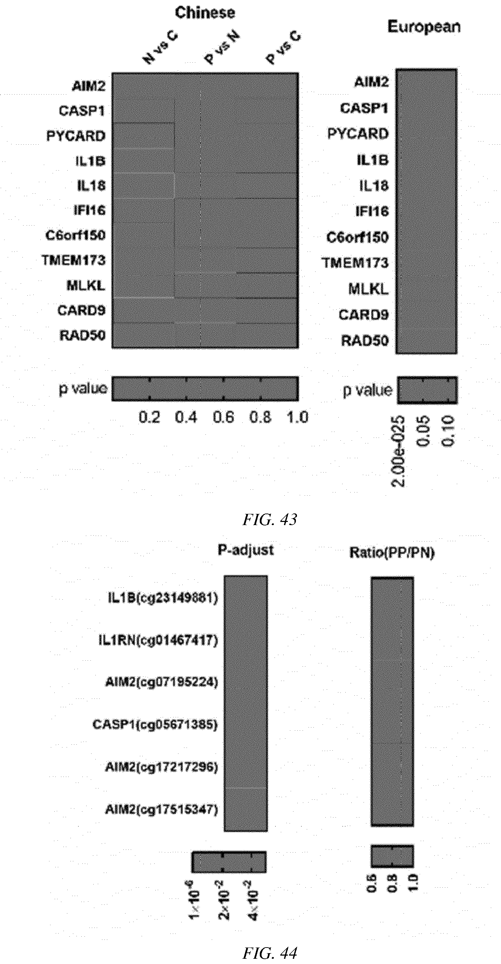

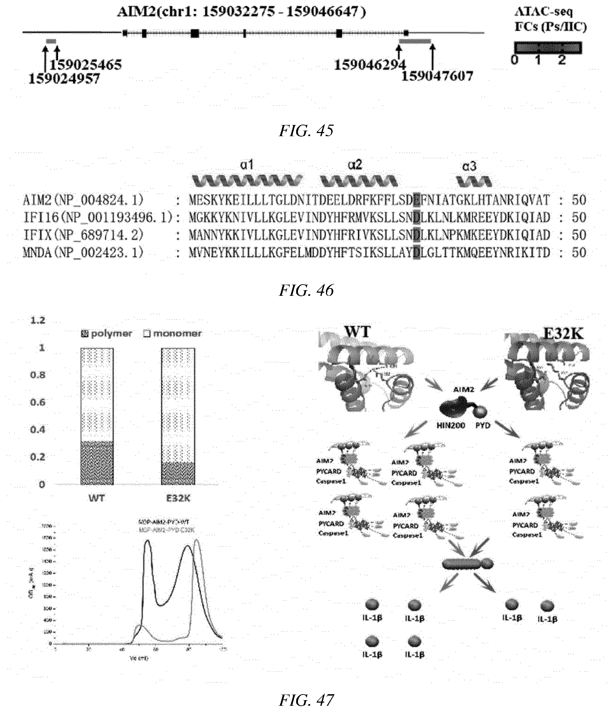

In this example of the present invention, the dsDNA-AIM2 pathway was found to be significantly associated with psoriasis by means of transcriptome sequencing (as shown in Figure 43), single-cell sequencing at transcription level, DNA methylation level detection (as shown in Figure 44) and ATAC sequencing (as shown in Figure 45). The inventors found that the conservative AIM2 E32K mutant has a protective effect against psoriasis through whole exome sequencing (as shown in Figure 46).

Subsequently, through bioinformatics prediction and *in vitro* protein expression polymerization experiment, this example of the present invention showed that the mutation of 32AA from an acidic amino acid to a basic amino acid affected the polymerization ability of AIM2 and weakened the active form of the polymer forming dsDNA-AIM2 (as shown in Figure 47), that is, weakened the function of this pathway and reduced inflammation response.

Finally, it should be noted that the above examples are only used to illustrate the technical solutions of the present invention and not to limit them. Although the present invention has been described in detail with reference to the preferred examples, those of ordinary skill in the art should understand that the technical solutions of the present invention can be modified or equivalently replaced without departing from the purpose and scope of the technical solutions of the present invention, and all of them shall be covered by the scope of the claims of the present invention.

## Claims

1. Use of an agent in the preparation of a medicine for treating/inhibiting psoriasis, **characterized in that** the agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

2. The use according to claim 1, **characterized in that** the agent is selected from IL-1β monoclonal antibodies.

3. The use according to claim 1, **characterized in that** the dosage form of the medicine for treating/inhibiting psoriasis is a dressing, an oral medicament, a subcutaneous injection, or an intravenous injection.

4. Use of an agent in the preparation of an inhibitor for IL17 secretion by γδ T cells, **characterized in that** the agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

5. Use of an agent in the preparation of a medicament for inhibiting the spread of skin tissue lesions, **characterized in that** the agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

6. Use of an agent in the preparation of an inhibitor for keratinocyte hyperproliferation in skin tissue, **characterized in that** the agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

7. Use of an agent in the preparation of an inhibitor for AIM2 inflammasome response, **characterized in that** the agent is selected from at least one of the following: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

8. A method for inhibiting IL17 secretion by γδ T cells *in vitro,* **characterized in that** the method comprises knocking out AIM2-expressing gene and/or IL1b-expressing gene and/or receptor IL1r-expressing gene in cells *ex vivo.*

9. A method for inhibiting AIM2 inflammasome response *in vitro,* **characterized in that** the method comprises using at least one of the following agents for interference: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.

10. A method for inhibiting keratinocyte hyperproliferation in skin tissue *in vitro,* **characterized in that** the method comprises using at least one of the following agents for interference: (1) interleukin 1b antagonists and biological activity inhibitors or functional analogues thereof; (2) dsDNA antagonists and biological activity inhibitors or functional analogues thereof or dsDNA degrading agents; (3) AIM2 antagonists and biological activity inhibitors or functional analogues thereof; (4) caspase1 antagonists and biological activity inhibitors or functional analogues thereof; (5) interleukin 1b expression inhibitors, dsDNA production inhibitors, AIM2 expression inhibitors or caspase1 expression inhibitors; (6) AIM2 polymerization inhibitors or dsDNA binding agents or functional analogues thereof; (7) Aim2 gene DNA methylation promoting agents; (8) interleukin 1r1 binding agents or interleukin 1r2 binding agents; (9) interleukin 1ra expression promoting agents; (10) interleukin 17a expression promoting agents or interleukin 17f expression promoting agents.
